(19) Europäisches Patentamt
European Patent Office
Office européen des brevets

(11) **EP 3 513 729 A1**

(12) **EUROPEAN PATENT APPLICATION**

(43) Date of publication:
**24.07.2019 Bulletin 2019/30**

(51) Int Cl.:
*A61B 6/00* (2006.01)    *A61B 6/06* (2006.01)

(21) Application number: **18152273.1**

(22) Date of filing: **18.01.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(71) Applicant: **Koninklijke Philips N.V.
5656 AE Eindhoven (NL)**

(72) Inventor: **SCHLUETER, Mathias
5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **N-IMAGE STITCHING WITH MINIMAL TRAVERSE PATH OF A FLAT PANEL DETECTOR**

(57)     An imaging system (100) for imaging an object (10) using a stitching sequence of partial images (311, 321) is disclosed comprising radiation generating means (200) and a detector (300), which can be moved independently. The imaging system (100) ensures that a region of X-ray in a first partial image (311) out of a stitching sequence of images touches an upper edge (341) of an active region (340) of the detector, and a region of X-ray in a last partial image (321) out of a stitching sequence of images touches a lower edge (342) of an active region of the detector.

Fig. 1

Printed by Jouve, 75001 PARIS (FR)

## Description

FIELD OF THE INVENTION

The invention relates to an imaging system for acquiring an image of an object using a stitching sequence of images, a method for acquiring an image of an object using a stitching sequence of images, a computer program element for controlling such imaging system for performing such method and a computer readable medium having stored such computer program element.

BACKGROUND OF THE INVENTION

In radiographic X-ray examinations of e.g. a full leg or a full spine, X-ray images may be required that exceed the height of a flat panel X-ray detector. For acquisition of such X-ray image covering a body part of interest, several sub-images or partial images of different parts of the body part can be obtained. These partial images can then be stitched together to a composite image using digital image processing techniques to create a full image of the body part. In order to obtain reliable stitching results with high image quality, subsequent partial images in the stitching sequence have to have a certain image region overlap.

There are two acquisition approaches to obtain a composite image by multiple image stitching. The first approach is a single focus approach. In this case the flat panel X-ray detector is moved and the X-ray tube is tilted between acquisitions of subsequent partial images. Consequently all partial images obtained in this case have the same focal point. It is desirable that the number of partial images to be acquired during acquisition of a stitching sequence is minimal for a given examination length and given overlap of partial images. In this way, for instance, a reduction of a radiation dose for a patient during X-ray examination can be achieved. Furthermore, the memory usage for storing and processing the partial images can be reduced. In case of the single focus approach, the issue of obtaining a minimal number of partial images could be addressed by larger vertical collimation sizes. However, if the vertical collimation size becomes large, heel effect or r2-decay artifacts may occur in overlap regions between images, especially for large tilt angles. Therefore, the collimation size should be configurable in order to find a compromise between image quality and patient dose.

A second approach in multiple image stitching is a parallel stitching approach, where the focal point is moved by lateral movement of the X-ray tube between acquisitions of subsequent partial images in a stitching sequence. This approach suffers from parallax errors which may be visible in overlapping areas of subsequent partial images. In order to keep the parallax error small, the collimation size should be small. On the other hand, from a patient dose and memory point of view, the total number of partial images should be minimal. Therefore, also in the parallel stitching approach, the collimation size should be configurable in order to find a compromise between image quality and patient dose.

In both approaches, the available free path length for the flat panel X-ray detector may be limited due to geometrical constraints in e.g. an examination room in a hospital, while a maximal body part coverage of an object to be examined is desired. In this context, a free path length for the X-ray detector refers to the path or way along which the detector can be moved or relocated during image acquisition.

The image acquisition within multiple image stitching may therefore still be improved.

SUMMARY OF THE INVENTION

Hence, there may be need to provide an imaging system for acquiring a composite image using a stitching sequence of sub-images or partial images with an improved image acquisition.

The object of the present invention is solved by the subject-matters of the independent claims, wherein further embodiments are incorporated in the dependent claims. It should be noted that the aspects of the invention described in the following apply also to the imaging system, the method, the computer program element, and the computer readable medium.

According to an aspect of the present invention, an imaging system for acquiring an image of an object using a stitching sequence of partial images is presented. The imaging system comprises radiation generating means and a detector. The radiation generating means and the detector are configured for being moved independently with respect to each other during a generation of the stitching sequence. Thereby, an independent movement of the radiation generating means and the detector may be realized by means for moving the radiation generating means and means for moving the detector, respectively. The latter means may be controlled independently and may move the radiation generating means and the detector, respectively. An independent movement may be realized by different starting times for a respective movement, by different accelerations, different acceleration times, acceleration paths, and/or by different velocities of the radiation generating means and the detector. The radiation generating means may be e.g. moved or e.g. rotated, while at the same time, the detector may be at rest. Similarly, the radiation generating means may be at rest and the detector may be moved by acceleration or uniform velocity. Also, the detector and the radiation generating means can move at the same time with different velocities and/or accelerations. The radiation generating means and the detector may be moved automatically, e.g. by means of an electronic drive, but also a manual movement initiated by medical staff may be possible. A stitching sequence comprises several sub-images or partial images, each sub-image or partial image covering a part of an object to be imaged. The imaging system is configured for ensuring that the region of X-ray in the first partial image of the stitching sequence in the first detector position starts at the upper edge of the active region of the detector. In other words, the imaging system is configured to ensure that the region of X-ray in the first partial image within a series of stitching images touches the upper edge of the active region of the detector. The region of X-ray, or, in other words, the collimation area, is the region on the active region of the detector, which is exposed to the X-ray radiation. In other words, the region of X-ray in a partial image on the detector is the region with a non-vanishing X-ray intensity, i.e. an area hit by the X-rays generated by the radiation generating means. Thereby, positions of the detector, the active region of the detector as well as the radiation generating means may be determined, e.g. by using sensors or by any other determination of the spatial positions. From the determined positions, new positions may be calculated for which the region of X-ray in the first partial image within a series of stitching images touches the upper edge of the active region of the detector - if this has not yet been the case previously. The detector and/or the radiation generating means may then be moved to the calculated positions. Such movement may be an automatic movement, it may also be a manual movement by medical staff, wherein the positions ensuring that the region of X-ray in the first partial image starts at the upper edge of the detector's active region are displayed or otherwise communicated to an operator of the imaging system. The operator may then initiate a manual or an automatic movement to the respective positions. The imaging system may be configured to start with or to begin further operation only if the above condition - touching of the upper region of X-ray in a partial image with the upper edge of the active region of the detector - is fulfilled. The active region of the detector thereby allows detecting an intensity distribution of radiation transmitted through an object to be imaged. Hence, the active region of the detector can be realized by the region of the detector that is sensitive to X-ray radiation and allows for a measurement or determination of the intensity and/or spectral properties of the detected X-ray radiation. A partial image may be taken from the entire active region of the detector, or it may be taken from a region smaller than and contained in the active area of the detector.

Further, the imaging system is configured for ensuring that the region of X-ray in the last partial image of the stitching sequence in a last detector position ends at the lower edge of the active region of the detector. In other words, the imaging system is configured such that the region of X-ray in the last partial image acquired during a record of a stitching sequence and taken in the last detector position touches the lower edge of the active region of the detector. In addition, the system according to the first aspect of the invention is configured such that a collimation height of partial images of the stitching sequence is configurable.

The system may also be configured such that several collimation heights corresponding to different partial images in the stitching sequence are configurable. Thereby, there may be a display with an input mask, allowing an operator to input a collimation height or several collimation heights. An operator may be warned by an alert, that a collimation height is not yet configured. It may also be possible to choose a predefined value, e.g. stored default value, or to choose among several default values, which default values may be e.g. suitable for different imaging situations with differing object opacity, object size, or the like. The system may also be configured to calculate a suitable collimation height and to set the collimation height automatically to the calculated value.

A pre-configuration of the collimation height - or, in other words, the collimation size - for the partial images can be important in situations where the collimation height should be limited. In parallel stitching this is desired in order to keep a parallax error small. In single focus stitching with limited X-ray examination room size limiting the X-ray source to image distance (i.e. the X-ray tube to detector distance), the tilt angle of the X-ray generating means related to two successive positions of the radiation generating means, could be chosen to be relatively large, e.g. 30°. This may however lead to strong heel effects or $r^2$-decay artefacts. In order to reduce such artefacts within the partial images it is desirable to limit the collimation size.

By means of the above introduced radiation generating means, X-ray radiation can be generated. The radiation generating means can be embodied by an X-ray tube. The generated X-ray radiation may be collimated and radiate in direction of the detector. Such detector may be a flat panel X-ray detector or the like. The detector may detect X-ray radiation originally emitted by the radiation generating means in direction of the detector and potentially having traversed and being attenuated and/or deflected by an object to be imaged before reaching the active area of the detector. From detection of that radiation reaching the active area of the detector, a partial image of a stitching sequence can be obtained, respectively. From the partial images of the stitching sequence, a complete or full image of the object to be imaged and positioned in the beam path can be obtained by suitable image processing. The object can be e.g. a full leg or the full spine with a height or length larger than the length or height of the active area of the detector.

By ensuring, that the region of X-ray in the first partial image starts at or touches the upper end of the active region of the flat panel detector, and further ensuring that the region of X-ray in the last partial image ends at or touches the lower end of the flat panel detector's active region, the invention allows to minimize a traverse path of the detector for a given object length or, equivalently, a desired body part coverage. This, in turn, is equivalent to a maximal body part coverage for limited traverse path of the flat panel detector.

In an exemplary embodiment, the imaging system further comprises calculation means for calculating at least two positions of the radiation generating means and at least two detector positions. During acquisition of the stitching sequence, the radiation generating means and the detector can then be moved to the calculated positions. To this end, the imaging system further comprises means configured for causing an automatic motion of the radiation generating means, and configured for successively moving the radiation generating means to the at least two positions of the radiation generating means. Further, the imaging system comprises means configured for causing an automatic motion of the detector, for successively moving the detector to the at least two detector positions. Thereby, the automatic motion of the radiation generating means is independent of the automatic motion of the detector.

Hence, the invention allows, among others, to calculate positions of the radiation generating means and the detector in order to minimize a traverse path of the detector for given coverage of an object to be imaged.

According to an exemplary embodiment, the active region of the detector can be larger than the region of X-ray (ROX) on the detector. The region of X-ray is thereby characterized by a non-vanishing intensity distribution of radiation transmitted through the object to be examined. Hence, the region of X-ray on the detector is given by the part of the active region of the detector which is exposed to radiation during acquisition of a partial image, respectively. A partial image then comprises at least the region of X-ray on the detector. The area of the detector from which the partial image is acquired, however, may not necessarily be congruent to the detector's active area, but according to the invention, may be smaller than the detector's active area.

According to an exemplary embodiment, the motion of the radiation generating means is asynchronous to the motion of the detector. In other words, the velocities characterizing the radiation generating means' motion and the detector's motion may be different from each other. For instance, the detector may be moved faster than the radiation generating means or vice versa. This may be the case when the distance between subsequent positions of the detector is larger than the distance between subsequent radiation generating means' positions, or vice versa. It is also possible, that the detector is not but the radiation generating means are moved between acquisitions of two subsequent partial images within a stitching sequence, or vice versa. The aforementioned cases exemplarily describe an asynchronous motion of the radiation generating means and the detector with respect to each other. This can apply to single focus stitching, wherein the radiation generating means are tilted and the detector is moved along a traverse path, as well to parallel stitching, with movement of radiation generating means and detector along paths parallel to each other.

According to an exemplary embodiment, the motion of the radiation generating means is a linear motion. In this case, the embodiment of the imaging system of the present invention is configured to carry out parallel stitching.

According to an exemplary embodiment, the motion of the radiation generating means of the imaging system is a rotation. Hence, in this case, the embodiment of the imaging system of the present invention is configured to carry out single focus stitching.

According to an exemplary embodiment, the radiation generating means and the detector are moved simultaneously. During acquisition of a partial image, the radiation generating means as well as the detector may be at rest or move in order to minimize the acquisition time. When a subsequent position of radiation generating means and detector for acquisition of a subsequent partial image shall be taken, radiation generating means and detector may be moved at the same time to their respective new positions.

According to an exemplary embodiment, an overlap height between the region of X-ray in a preceding partial image and the region of X-ray in a subsequent partial image is configurable at the imaging system. The overlap height is of importance in the image processing that stitches the partial images together to the complete image of the object to be imaged. Such overlap refers to the overlap of regions of X-ray in subsequent partial images at one or several edges of the respective partial images. For instance, a partial image can overlap at its bottom edge with the top edge of a subsequent partial image. An overlap is essential in image processing for stitching partial images correctly together. Depending on the object, i.e. for example the part of the body to be imaged, a larger overlap height might be advantageous as otherwise it might be harder to stitch the partial images correctly together. This might be the case when the imaged structures do not show enough substructures that allow for an easy superposition of subsequent partial images at their respective corresponding edges. In other cases, e.g. where substructures of the object to be imaged are rich, a smaller overlap height can be chosen, also possibly and advantageously reducing the number of partial images that need to be taken to arrive at a complete image of the entire object to be imaged.

According to an exemplary embodiment, the imaging system further comprises image processing means. By the latter, all partial images acquired successively can be stitched together.

According to an exemplary embodiment, the imaging system is configured to move the detector such that a traverse path length of the detector is minimal. This is e.g. advantageous for limited examination room space.

According to an exemplary embodiment, a difference between a position of the radiation generating means and a detector position increases linearly during acquisition of the stitching sequence of images. This may refer to respective positions along a longitudinal axis, which longitudinal axis determines the direction along which both the detector and the radiation generating means are moved during acquisition of the stitching sequence. A transverse distance between detector and radiation generating means might not be considered in this context, but only the difference of positions in a projection onto a longitudinal axis.

According to an exemplary embodiment, a difference between a position of the radiation generating means and a detector position increases non-linearly during acquisition of the stitching sequence of images. Similarly to the aforementioned embodiment, respective positions along a longitudinal axis might be considered in this case, which longitudinal axis determines the direction along which both the detector and the radiation generating means are moved during acquisition of the stitching sequence.

In both the last two above embodiments, an increase of a difference between a detector position and a position of the radiation generating means implies, that the region of X-ray, i.e. the part of the active region of the detector, which experiences a non-vanishing X-ray intensity, is not the same for all partial images. It may even imply, that for two subsequent partial images, the region of X-ray on the detector differs. This idea of the invention is - among others - further elucidated in the exemplary embodiments.

According to a second aspect of the invention, a method for acquiring an image of an object using a stitching sequence of images is described. The method comprises the following steps, not necessarily in this order: in one step, radiation generating means and an X-ray detector are moved independently with respect to each other during a generation of the stitching sequence. In another step it is ensured that the region of X-ray in the first partial image of the stitching sequence in the first detector position starts at the upper edge of the active region of the detector. In yet another step it is ensured that the region of X-ray in the last partial image of the stitching sequence in the last detector position ends at the lower edge of the active region of the detector. Thereby, as already described in the context of the imaging system embodiments, a traverse path of the detector can be minimal for given body part coverage. In a further method step, a collimation height of partial images of the stitching sequence can be/is configured.

According to an exemplary embodiment, within the above described method, an overlap height between the region of X-ray in a preceding partial image and the region of X-ray in a subsequent partial image can be configured.

According to an exemplary embodiment, the above described method further comprises the following steps: in a step at least two positions of the radiation generating means and at least two detector positions are calculated. In another step an automatic motion of the radiation generating means is caused, and a successive motion of the radiation generating means to the at least two positions of the radiation generating means is caused. In yet another step an automatic motion of the detector, and a successive motion of the detector to the at least two detector positions, is caused. Thereby, the automatic motion of the radiation generating means is independent of the automatic motion of the detector. Further, the detector comprises an active region for detecting an intensity distribution of radiation transmitted through the object.

It is understood, that embodiments described in context of the imaging system can also be incorporated into method steps and vice versa without departing the scope of the present invention. In particular, a method supplemented by steps derived from exemplary embodiments related to the imaging system are also part of the present invention.

According to a third aspect of the invention, a computer program element for controlling an imaging system is presented, wherein the computer program element, when being executed by a processing unit, is adapted to perform the method as defined in the above second aspect of the invention and its corresponding exemplary embodiments.

These and further aspects of the present invention will become apparent from and be elucidated with reference to the embodiments described hereinafter.

BRIEF DESCRIPTIONS OF THE DRAWINGS

Fig. 1 shows a schematic diagram illustrating an embodiment of the imaging system according to the present invention;

Fig. 2 shows a schematic diagram showing the position of a region of X-ray in a first partial image and the position of a region of X-ray in a last partial image on a detector with active region according to an exemplary embodiment of the invention;

Fig. 3 shows a schematic diagram of an example of an imaging system according to the invention;

Fig. 4 shows a schematic diagram illustrating aspects of an exemplary embodiment according to the present invention;

Fig. 5 shows basic steps of an example of a method for imaging an object.

DETAILED DESCRIPTION OF EMBODIMENTS

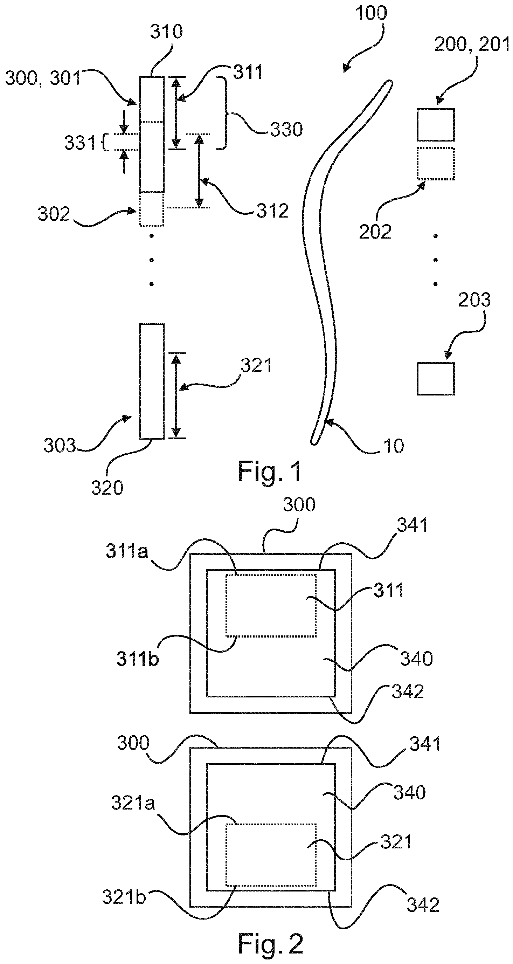

Fig. 1 shows schematically and exemplarily an embodiment of an imaging system 100 for imaging an example object 10 according to the present invention. The example object 10 in Fig. 1 schematically shows a spine 10, which is positioned between radiation generating means 200 and a detector 300. The radiation generating means 200 may be realized by an X-ray tube and the detector 300 may be provided by a flat panel detector. It should be noted that the (relative) distances and positions of object 10 and X-ray tube 200 as well as the flat panel detector 300 are not to scale. However, in any case and as can be also seen in the schematic drawing, the object's 10 extension shall exceed the height of the detector 300, such that in order to acquire a full image of the object 10, several partial images have to be obtained and afterwards stitched together to a composite or full image. The rectangular with reference number 201 shows the X-ray tube 200 in a first position, while the dotted rectangular 202 refers to a second position of the X-ray tube. A last position of the X-ray tube is shown with reference number 203. Similarly, the flat panel detector 300 is shown in several position. The first position is indicated with reference number 301, while reference numbers 302 and 303 refer to the second and to the last position of the detector, respectively. Accordingly, both the X-ray tube and the detector can be moved, whereas the respective movement is - according to the present invention - independent from each other. An independent movement of the radiation generating means and the detector may be provided by means for moving the radiation generating means and means for moving the detector, respectively. An independent control of the latter means may be provided, allowing for independent movement of the radiation generating means and the detector, respectively. An independent movement may be realized by different starting times for a respective movement, by different accelerations, different acceleration times, acceleration paths, and/or by different velocities of the radiation generating means and the detector. The radiation generating means may be e.g. moved or e.g. rotated, while at the same time, the detector may be at rest. Similarly, the radiation generating means may be at rest and the detector may be moved by acceleration or uniform velocity. The radiation generating means and the detector may be moved automatically, e.g. by means of an electronic drive, but also a manual movement initiated by medical staff may be possible.

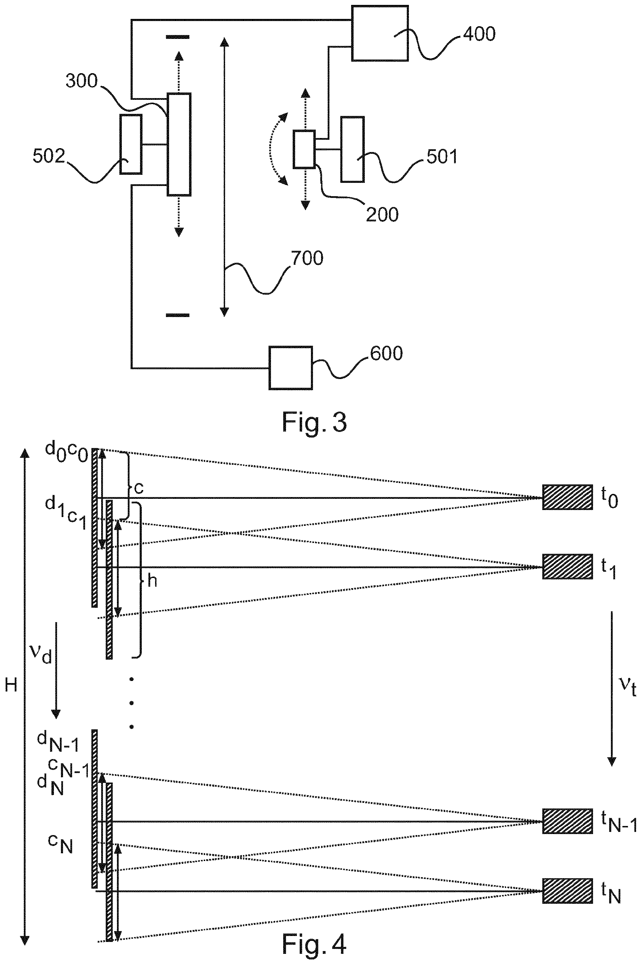

It can be seen from Fig. 1 that the height of the region of X-ray in a partial image 311, 312, 321, is smaller than the height of the flat panel detector. The latter height is given by the vertical distance between the upper edge 310 and the lower edge 320 of the detector. According to the invention, the collimation height of the region of X-ray may be preconfigured or is configurable. Further, the region of X-ray 311 in the first partial image and the region of X-ray 312 in the second partial image overlap in Fig. 1, with an overlap height 331 or in other words an overlap size 331. Such overlap of subsequent regions of X-ray in partial images is required for subsequently stitching the partial images acquired during a stitching sequence together by suitable image processing means. According to the present invention, an upper region of the region of X-ray 311 in the first partial image touches or starts at the upper edge 310 of the detector, while a lower region of the region of X-ray 321 in the last partial image touches or ends at the lower edge 320 of the detector. The overlap height 331 may be preconfigured and the upper and the lower position of the object 10 to be imaged have to be determined prior to the acquisition of the stitching sequence. Based on the latter information, according to the invention, a minimal required number of images may be calculated. Calculation may be carried out by calculation means of the imaging system. However, there may also be external calculation means, which carry out the calculation and transfer the calculated result to the imaging system by wired or wireless connection. Also a user may perform such calculation and input the result manually. Further and optionally, a collimation size for the regions of X-ray in the partial images and the subsequent X-ray tube and detector positions may be determined. For parallel stitching the collimation height should be chosen not larger than of e.g. 6 cm. For a focal point to detector distance of 180 cm, this leads to acceptable parallax errors. For single focus stitching the collimation height can be between half and full detector height, e.g., between 21 cm and 42 cm. An exemplary determination of a collimation size is described in context of Fig. 4. The latter aspects will be further discussed in context of Fig. 3, 4 and Fig. 5.

Fig.2 shows two plan views on the flat panel detector 300, one in its first position 301 and one in its last position 303, respectively. The detector has an active area 340. The active area 340 shown in Fig. 2 is rectangular with an upper edge 341 and a lower edge 342. Within the active area 340, the dotted rectangular shows the region of X-ray 311 of the first partial image and the region of X-ray 321 in the last partial image 321, respectively. An upper region of X-ray in the first partial image 311 with upper edge 311a touches the upper edge 341 of the active region 340 of the detector. However, the lower edge 311b of the region of X-ray in the first partial image 311 lies within the active region 340 of the detector and does not reach to or touch the lower edge 342 of the active region 340. In Fig. 2, exemplarily, the lower edge 311b of the region of X-ray in the first partial image 311 lies horizontally slightly above the middle of the active area 340. Similarly, the region of X-ray in the last partial image 321 with lower edge 321b touches or reaches to the lower edge 342 of the active area 340 of the detector. The upper edge 321a of the region of X-ray in the last partial image 321, however, lies within the active area 340 and does not reach to or touch the active area's upper edge 341. In this way, a minimal traverse path of the flat panel detector can be achieved while simultaneously allowing a maximal body part coverage.

Fig.3 schematically shows an imaging system according to an embodiment of the present invention, with radiation generating means and a collimator (both components indicated in Fig. 3 by reference sign 200), wherein the radiation generating means may be given by an X-ray tube, and a flat panel detector 300. The X-ray tube and the collimator 200 are attached to motion means 501, which may cause an automatic movement of the X-ray tube and the collimator 200. The collimator may restrict the radiation generated by the X-ray tube before the radiation passes through an object to be imaged and is subsequently detected at the detector. With the collimator the collimation height can be configured or determined. The motion means 501 may further successively move the X-ray tube and collimator 200 to at least two different positions. A movement of the X-ray tube may be a rotation, indicated by the dotted arc segment with arrows. Such movement refers to an embodiment related to single focus stitching. In case of parallel stitching, a movement of the X-ray tube and collimator is a lateral movement, which is indicated by the dotted lines with arrows up and down.

The detector 300 is attached to motion means 502, which allow an automatic and lateral movement of the detector. The motion means 502 allow for a successive movement of the detector 300 to at least two different positions. In both cases of parallel and single focus stitching, the movement of the flat panel detector 300 is a lateral one, which is indicated by the dotted arrows in the schematic drawing of Fig. 3, attached to the detector 300. According to the invention, the flat panel detector 300 shall be moved such, that its active area is successively moved along or covers the length 700 of the object to be imaged (not shown in Fig. 3). The detector is connected to image processing means 600. The latter image processing means 600 allow for stitching together the partial images in an acquired stitching sequence to a complete composite image of the object. Such connection between detector 300 and image processing means 600 may be a wired or wireless connection. Further, both the detector 300 and the X-ray tube and collimator 200 are connected to calculation means 400, whereas the connection may be wired or wireless. The calculation means 400 allow for a calculation of at least two positions of the detector and at least two positions of the X-ray tube. Thereby, the calculation means determine the first position of detector and X-ray tube such, that a region of X-ray in a first partial image of the stitching sequence in the first detector position starts at or touches the upper edge of the active region of the detector, compare to Fig. 2. Similarly, the calculation means determine the last detector and X-ray tube positions such, that the region of X-ray in the last partial image's region of the stitching sequence in the last detector position ends at the lower edge of the active region of the detector. The calculated positions are transferred to the detector and X-ray tube and the motion means 501 and 502 may then cause a respective movement to the calculated positions. Further and optionally, a collimation height for partial images may be configured, e.g. by a manual input of a user, which manual input may be transferred to the calculation means. Also, the collimation height may be calculated in case a minimal number of partial images is desired. The calculated or manually input collimation height may then be transferred to the collimator and the latter may be adjusted to the collimation height.

Fig.4 serves to illustrate a calculation of a required number N of partial images according to an embodiment of the invention. Thus, the imaging system may be configured to perform such a calculation. E.g. a calculation unit of the imaging system may be configured to perform the calculations described in the following. In Fig. 4, $H$ denotes the height of the object, e.g. the body part, to be imaged, and $h$ is the height of a detector's active region. With c a collimation height is indicated, $o_d$ and $o_c$ denote an overlap height between subsequent detector and collimation positions, respectively. The aforementioned heights may be determined, e.g. measured by sensors of the system, and communicated internally by the system, or heights might be otherwise known to the operator - e.g. by manufacturer specifications or user's guides and may be input in e.g. an input mask at the imaging system. As, according to the invention, the exposure for the first partial image is such that the top edge of the detector's active region is touched, and the exposure for the last partial image is such that the bottom edge of the detector's active region is touched, two equations for the height of the object may be derived:

1. $H = N h - (N - 1) o_d$
2. $H = N c - (N - 1) o_c$

From the second equation, a minimal number N of images can be derived:

$$N >= (H - o_c)/(h - o_c).$$

If we choose $N_{min}$ as the smallest integer number fulfilling this inequality, a maximal collimation height may be calculated from equation 2 as

$$c_{max} = (H + (N_{min} - 1) o_c)/N_{min}.$$

As in case of parallel stitching or single focus stitching with relatively large X-ray tube tilt, the collimation height should be limited, the collimation height c should be configured in addition to the overlap height $o_c$. Then, a lower bound for the number of partial images can be calculated by the imaging system from equation 2 as

$$N >= (H - o_c)/(c - o_c).$$

With further reference to Fig. 4, if the origin of the coordinate system is put at the top left corner of the detector in the first detector position, the subsequent detector and collimation position for partial image n will be given by

$$d_n = n (h - o_d),$$

$$c_n = n (c - o_c).$$

From equation 1 and 2 above, detector and collimation overlap heights are obtained as

$$o_d = (N\,h - H)/(N - 1),$$

$$o_c = (N\,c - H)/(N - 1).$$

Consequently, one obtains the collimation positions relative to the detector positions, $C_n$:

$$C_n = c_n - d_n = n\,(h - c)/(N - 1).$$

In this example, the collimation position - and hence the position of the X-ray tube in longitudinal direction - relative to the detector position increases linearly with the partial image number n. In other embodiment, the difference between a position of the X-ray tube and a detector position may increase non-linearly during acquisition of a stitching sequence. For the first and last relative collimation position in the example of Fig. 4, one has:

$$C_0 = 0,\ C_N = h - c.$$

This implies that the top and bottom collimation border for the first and last partial image is at the top and bottom border of the detector, respectively. This in turn implies a minimal traverse path length of the detector, which leads to a maximal possible body part height for an X-ray system with certain geometrical constraints. It should be noted further, that the tube positions $t_n$ for parallel stitching can be determined to be:

$$t_n = c_n + c/2,$$

whereas the tilt angle is fixed to zero with respect to the detector normal. In single focus stitching, the tube position is fixed to $t = H/2$.

The tilt angles $a_n$ with respect to the detector normal are approximately given by:

$$\tan(\alpha_n) \approx (t - c_n - c/2) \,/\, SID,$$

where SID is the distance of the focal point of the X-ray tube to the detector.

The imaging system may then, with calculated detector and collimation positions for all images within the stitching sequence of images, initiate a stitching sequence.

Due to patient motion during acquisition of a stitching sequence, the total time for a stitching acquisition should be as small as possible. Therefore, a continuous detector and tube motion is desirable. This can be realized for a fluoroscopy X-ray system, which acquires images with a certain frame rate. Then, for parallel stitching, the tube and detector motion can be calculated in the following way: The differences between subsequent detector and collimation positions are given by

$$D = d_{n+1} - d_n = (H - h)/(N - 1),$$

$$C = c_{n+1} - c_n = (H - c)/(N - 1).$$

The latter do not depend on the partial image number. For parallel stitching, the collimation position differences are equal to the tube position differences. Then, the detector and tube velocity are derived as the product of the respective detector or collimation position difference with the frame rate $f$:

$$v_d = f\,(H - h)/(N - 1),$$

$$v_t = f\,(H - c)/(N - 1).$$

The above in context of Fig. 4 described calculation may be carried out by the calculation unit according to the present invention. Similar calculation steps may be carried out for the case of single focus stitching, where the X-ray tube is rotated instead moved along a linear path.

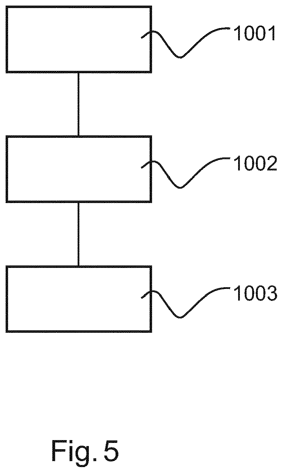

Fig.5 shows an example of a method according to the present invention. The method comprises the following three steps: In step 1001, an X-ray tube and a detector are moved independently with respect to each other during a generation of a stitching sequence. In step 1002, it is ensured by both calculation means and means for moving the detector and means for moving the X-ray tube and means for collimating the radiation that a region of X-Ray in a first partial image of the stitching sequence in a first detector position starts at an upper edge of an active region of the detector. Finally, in step 1003 it is ensured by calculation means and means for moving detector, X-ray tube and collimator, respectively, that a region of X-ray in a partial image of the stitching sequence in a last detector position ends at the lower edge of the active region of the detector. Within the method it is further comprised, that a collimation height of regions of X-ray within the partial images of the stitching sequence is configurable. Such configuration may be achieved by e.g. a manual input of a user. Further, an overlap height between a region of X-ray in a preceding partial image and a region of X-ray in a subsequent partial image may also be configurable.

It has to be noted that embodiments of the invention are described with reference to different subject matters. In particular, some embodiments are described with reference to method type claims whereas other embodiments are described with reference to device type claims, referring to an imaging system. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of features.

While the invention has been illustrated and described in detail the drawings and the foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items recited in the claims. The mere fact that certain measures are re-cited in mutually different dependent claims does not indicate that a combination of these measures cannot be used to advantage. Any reference signs in the claims should not be construed as limiting the scope.

**Claims**

1. An imaging system (100) for acquiring an image of an object using a stitching sequence of images, the imaging system comprising:

   radiation generating means (200),
   a detector (300),
   wherein the radiation generating means and the detector are configured for being moved independently with respect to each other during a generation of the stitching sequence,
   wherein the imaging system is configured for ensuring that a region of X-ray in a first partial image (311) of the stitching sequence in a first detector position (301) starts at an upper edge (341) of an active region (340) of the detector,
   wherein the imaging system is configured for ensuring that a region of X-ray in a last partial image (321) of the stitching sequence in a last detector position (303) ends at the lower edge (342) of the active region of the detector, and
   wherein a collimation height (330) of partial images of the stitching sequence is configurable.

2. Imaging system (100) according to claim 1 further comprising:

   calculation means (400) for calculating at least two positions of the radiation generating means and at least two detector positions,
   means (501) configured for causing an automatic motion of the radiation generating means, and configured for successively moving the radiation generating means to the at least two positions of the radiation generating means,
   means (502) configured for causing an automatic motion of the detector, for successively moving the detector to the at least two detector positions,
   wherein the automatic motion of the radiation generating means is independent of the automatic motion of the detector.

3. Imaging system according to claim 2, wherein the active region (340) of the detector can be larger than a region of X-ray in a partial image on the detector, the region of X-ray being **characterized by** a non-vanishing intensity distribution of radiation transmitted through the object.

4. Imaging system according to claim 2 or 3, wherein the motion of the radiation generating means is asynchronous to the motion of the detector.

5. Imaging system according to one of the preceding claims, wherein the motion of the radiation generating means is a linear motion.

6. Imaging system according to one of the preceding claims, wherein the motion of the radiation generating means is a rotation.

7. Imaging system according to one of the preceding claims, wherein the radiation generating means and the detector are moved simultaneously.

8. Imaging system according to one of the preceding claims, wherein an overlap height (331) between a region of X-ray in a preceding partial image and a region of X-ray in a subsequent partial image is configurable at the imaging system.

9. Imaging system according to the preceding claim, the imaging system further comprising image processing means (600), wherein all partial images acquired successively are stitched together by the image processing means.

**10.** Imaging system according to one of the preceding claims,
wherein the imaging system is configured to move the detector such that a traverse path length of the detector is minimal.

**11.** Imaging system according to one of the preceding claims,
wherein a difference between a detector position (301, 302, 303) and a position of the radiation generating means (201, 202, 203) increases linearly during acquisition of the stitching sequence of images.

**12.** Imaging system according to one of claims 1 to 10,
wherein a difference between a detector position and a position of the radiation generating means increases non-linearly during acquisition of the stitching sequence of images.

**13.** A method for acquiring an image of an object using a stitching sequence of images, the method comprising the following steps:

moving radiation generating means and a detector independently with respect to each other during a generation of the stitching sequence (1001),
ensuring that a region of X-Ray in a first partial image of the stitching sequence in a first detector position starts at an upper edge of an active region of the detector (1002),
ensuring that a region of X-Ray in a last partial image of the stitching sequence in a last detector position ends at the lower edge of the active region of the detector (1003),
wherein a collimation height of partial images of the stitching sequence is configurable,
wherein an overlap height between a region of X-ray in a preceding partial image and a region of X-ray in a subsequent partial image is configurable.

**14.** A method according to claim 13, further comprising the steps:

calculating at least two positions of the radiation generating means and at least two detector positions,
causing an automatic motion of the radiation generating means, and successively moving the radiation generating means to the at least two positions of the radiation generating means,
causing an automatic motion of the detector, and successively moving the detector to the at least two detector positions,
wherein the automatic motion of the radiation generating means is independent of the automatic motion of the detector;

wherein the detector has an active region for detecting an intensity distribution of radiation transmitted through the object.

**15.** Computer program element for controlling an imaging system according to one of the claims 1 to 12, which, when being executed by a processing unit, is adapted to perform the method steps of one of the claims 13 or 14.

Fig. 1

Fig. 2

Fig. 3

Fig. 4

Fig. 5

Europäisches
Patentamt

European
Patent Office

Office européen
des brevets

# EUROPEAN SEARCH REPORT

Application Number

EP 18 15 2273

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/038454 A1 (MINNIGH TODD R [US] ET AL) 17 February 2011 (2011-02-17) * paragraph [0028] - paragraph [0084]; figure 5 * | 1-15 | INV. A61B6/00 A61B6/06 |
| X | JP 2010 227372 A (SHIMADZU CORP) 14 October 2010 (2010-10-14) * paragraph [0045] - paragraph [0084]; figures 1-7 * | 1-15 | |
| X | US 2015/250441 A1 (OKUNO TOMOHARU [JP] ET AL) 10 September 2015 (2015-09-10) * paragraph [0018] - paragraph [0084]; figures 1-10 * | 1-15 | |
| X | JP 2007 260027 A (HITACHI MEDICAL CORP) 11 October 2007 (2007-10-11) * paragraph [0006] - paragraph [0046]; figures 1-12 * | 1-15 | |
| X | US 2011/206185 A1 (SAKAI TAKIHITO [JP] ET AL) 25 August 2011 (2011-08-25) * paragraph [0014] - paragraph [0071]; figures 1-12 * | 1-15 | TECHNICAL FIELDS SEARCHED (IPC) A61B |
| X | US 2004/247081 A1 (HALSMER MATTHEW A [US] ET AL) 9 December 2004 (2004-12-09) * paragraph [0017] - paragraph [0057]; figures 1-4 * * paragraph [0066] - paragraph [0100] * | 1-15 | |

The present search report has been drawn up for all claims

| Place of search | Date of completion of the search | Examiner |
|---|---|---|
| Munich | 12 June 2018 | Martinez Möller, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 15 2273

This annex lists the patent family members relating to the patent documents cited in  the above-mentioned European search report.
The members are as contained in the European Patent Office EDP file on
The European Patent Office is in no way liable for these particulars which are merely given for the purpose of information.

12-06-2018

| Patent document cited in search report | | Publication date | Patent family member(s) | | Publication date |
|---|---|---|---|---|---|
| US 2011038454 | A1 | 17-02-2011 | NONE | | |
| JP 2010227372 | A | 14-10-2010 | JP | 5574078 B2 | 20-08-2014 |
| | | | JP | 2010227372 A | 14-10-2010 |
| US 2015250441 | A1 | 10-09-2015 | CN | 104717923 A | 17-06-2015 |
| | | | EP | 2904973 A1 | 12-08-2015 |
| | | | JP | 6102935 B2 | 29-03-2017 |
| | | | JP | WO2014054417 A1 | 25-08-2016 |
| | | | US | 2015250441 A1 | 10-09-2015 |
| | | | WO | 2014054417 A1 | 10-04-2014 |
| JP 2007260027 | A | 11-10-2007 | JP | 4740779 B2 | 03-08-2011 |
| | | | JP | 2007260027 A | 11-10-2007 |
| US 2011206185 | A1 | 25-08-2011 | CN | 102076261 A | 25-05-2011 |
| | | | JP | 5549595 B2 | 16-07-2014 |
| | | | JP | WO2010050032 A1 | 29-03-2012 |
| | | | US | 2011206185 A1 | 25-08-2011 |
| | | | WO | 2010050032 A1 | 06-05-2010 |
| US 2004247081 | A1 | 09-12-2004 | EP | 1484016 A1 | 08-12-2004 |
| | | | EP | 1484017 A1 | 08-12-2004 |
| | | | JP | 4458936 B2 | 28-04-2010 |
| | | | JP | 4545490 B2 | 15-09-2010 |
| | | | JP | 2004358254 A | 24-12-2004 |
| | | | JP | 2004358255 A | 24-12-2004 |
| | | | US | 2004247081 A1 | 09-12-2004 |
| | | | US | 2005169427 A1 | 04-08-2005 |

EPO FORM P0459

For more details about this annex : see Official Journal of the European Patent Office, No. 12/82